# EUROPEAN PATENT APPLICATION

(11) **EP 4 407 144 A1**
(43) Date of publication of application: **31.07.2024**
(21) Application number: 23153979.2
(22) Date of filing: 30.01.2023
(51) Int. Cl.: E21B 49/00, G01N 21/63

(54) **DEVICE AND METHOD FOR OBTAINING GEOLOGICAL INFORMATION OF A SOIL LAYER**

(71) Applicant: Royal Eijkelkamp B.V., 6987 EN Giesbeek (NL)
(72) Inventor: Eijkelkamp, Hugo Jaap, 6987 EN Giesbeek (NL); Lichtenberg, Marcus Hermanus, 7231 LC Warnsveld (NL)
(74) Representative: 't Jong, Bastiaan Jacob

(57) **Abstract**

The invention relates to a device for obtaining geological and/or chemical information of a soil layer, which device comprises
- a string of driving rods; and
- a head arranged at the end of the string of driving rods, such that the head can be driven into a soil layer by the string of driving rods,
which head comprises an elongate, preferably cylindrical, body having on one elongate end a penetration cone and having on the other elongate end a coupling, such as a threaded end, for coupling with the string of driving rods
- a device for laser-induced breakdown spectroscopy and / or Raman spectroscopy having at least a laser device for generating a laser beam to be directed on to a material, an optical path for receiving light emitted from the material, and a spectrometer for separating and measuring spectral components of the received light transported along the optical path;
wherein the laser beam exits the device at the head and wherein the optical path starts at least adjacent to the position where the laser beam exits the head.

## Description

The invention relates to a device for obtaining geological and/or chemical information of a soil layer, which device comprises
- a string of driving rods; and
- a head arranged at the end of the string of driving rods, such that the head can be driven into a soil layer by the string of driving rods,
which head comprises an elongate, preferably cylindrical, body having on one elongate end a penetration cone and having on the other elongate end a coupling, such as a threaded end, for coupling with the string of driving rods.

Such a device is typically used with cone penetration testing. A driving rig is provided at the surface on a soil layer and the head is driven into the soil with the string of driving rods and the driving rig. By monitoring the depth of the head and the force required to advance the head into the soil layer, information on the geological and/or chemical structure of the soil layer is obtained. For example, clay can be more easily be penetrated than a rock like layer. And a sand layer is more easily penetrated than a clay layer. The force required to push the head through such a type of material is thus indicative for the type of material.

In order to obtain more detailed geological information of a soil layer, it is for example known from US 7692789 to drive a soil sample tube into a soil layer, retract the whole tube from the soil. The tube with the soil sample is transported to a laboratory and then the removed soil sample is examined with a laser-induced breakdown spectroscopy (LIBS)/ and/or Raman device to determine the elements in the soil sample at the different discrete positions.

When the soil sampling tube together with the soil sample is to be transported off site to a laboratory, it is essential to preserve the soil sample as best as possible. For example, any moisture has the tendency to evenly distribute through the sample, especially when the pressure of surrounding soil is no longer present. The same applies for the consistency of the soil sample along the length of the sample. Loose layers would typically fall easily apart during transport and layers, which were pressurized by surrounding layers, can easily expand.

Furthermore, if at a specific site a number of soil samples are to be retrieved, then an equal number of soil sampling tubes is required. All of these sampling tubes need to be transported, which will increase costs substantially, apart from the specialized conditioning equipment required to condition each and every soil sample. However, this is not done in practice. Typically, the sample is just removed from the soil sampling tube and as a result the sample is completely disturbed.

It is the object of the invention to reduce or even remove the above-mentioned disadvantages.

This object is achieved according to the invention with a device according to the preamble, which is characterized by a device for laser-induced breakdown spectroscopy and / or Raman spectroscopy having at least a laser device for generating a laser beam to be directed on to a material, an optical path for receiving light emitted from the material, and a spectrometer for separating and measuring spectral components of the received light transported along the optical path;
wherein the laser beam exits the device at the head and wherein the optical path starts at least adjacent to the position where the laser beam exits the head.

By having a device for laser-induced breakdown spectroscopy and / or Raman spectroscopy being part of the device for obtaining geological information of a soil layer and by having the laser beam exiting the device at the head and the optical path starting at least adjacent to the position where the laser beam exits the head, it is possible with the device according to the invention to obtain the geological and/or chemical information in situ. So, the soil can stay in the ground such that moisture, pressure, temperature and oxygen level remain the same at the moment the soil directly next to the head is examined. With laser-induced breakdown spectroscopy it is possible to detect chemical elements, such as carbon, potassium and manganese, while with Raman spectroscopy, it is even possible to detect chemical compounds. This also includes biological matter, which is composed out of chemical compounds.

The device also allows for a faster determination of the geological information of a soil layer at the penetration location, as it no longer requires retraction of a soil sample and transportation to a laboratory.

Furthermore, only a single device is required to make multiple penetrations over an area in order to determine how specific properties of the soil layer spread over said area.

In a preferred embodiment of the device according to the invention the position where the laser beam exits the head is on the elongate circumferential surface.

Having the laser beam exiting the on the elongate circumferential surface, i.e. in case of a cylindrical body in radial direction, ensures that the exit point of the laser is not subjected to the large forces to which the penetration cone is subjected. It also ensures that soil parts will be wiped away from the exit point of the laser and start of the optical path and will not be collected as is more likely at the penetration cone.

In a further preferred embodiment of the device according to the invention the optical path comprises an optical fiber starting at the head and running through the string of rods.

Using optical fibers allows for the more costly parts of the device, in particular the spectrometer to be positioned on the surface of the soil layer, while the head and the driving rods are driven into the soil layer. When the head gets stuck into the soil layer and cannot be retrieved, the spectrometer can still be used with a replacement head.

Preferably, the laser beam is guided along the optical fiber towards the head. Having the laser beam also traveling along the optical fiber allows the laser device to be positioned at the surface of the soil layer. Typically, the laser beam can be injected into the optical fiber by an angled two-way mirror, such that the received light emitted from the material can travel in the opposite direction and be fed to the spectrometer.

In yet another embodiment of the device according to the invention a force sensor is arranged between the penetration cone and the body. Monitoring the force with which the cone is advanced into the soil layer provides additional information on the geology of the soil layer.

The invention further relates to a method for obtaining geological information of a soil layer, which method comprises the steps of:
- providing a driving rig positioned on the surface of a soil layer;
- providing the device according to the invention;
- driving the head of the device with the string of driving rods into the soil layer by driving the string of driving rods with the driving rig;
- while monitoring the depth of the head into the soil layer repeatedly operating the device for laser-induced breakdown spectroscopy and / or Raman spectroscopy and registering the measured spectral components in combination with the depth of the head.

The method according to the invention allows for a quick determination of geological information of a soil layer. While driving the head of the device into the soil layer, measurements can be simultaneously made, such that when a desired depth is reached, also the desired measurements are available. With the method according to the prior art, soil samples needed to be removed from the soil layer and transported to a laboratory, such that a considerable amount of time passes before the geological information is available.

In a preferred embodiment of the method according to the invention, in combination with a force sensor arranged between the body and the penetration cone, the registered force of the force sensor is monitored and registered in combination with the depth of the head.

By also registering the force of the force sensor, additional information is available, which can help by interpreting the measured spectral components or helps in determining drilling parameters when after the measurement a hole is to be drilled.

These and other features of the invention will be elucidated in conjunction with the accompanying drawings.
Figure 1 shows a schematic cross section of a first embodiment of a device according to the invention.
Figure 2 shows a schematic cross section of a second embodiment of a device according to the invention.
Figure 3 shows a schematic view of an embodiment of the method according to the invention.
Figure 4 show schematically the functionality of the device of figure 1.
Figure 5 show schematically the functionality of the device of figure 2.

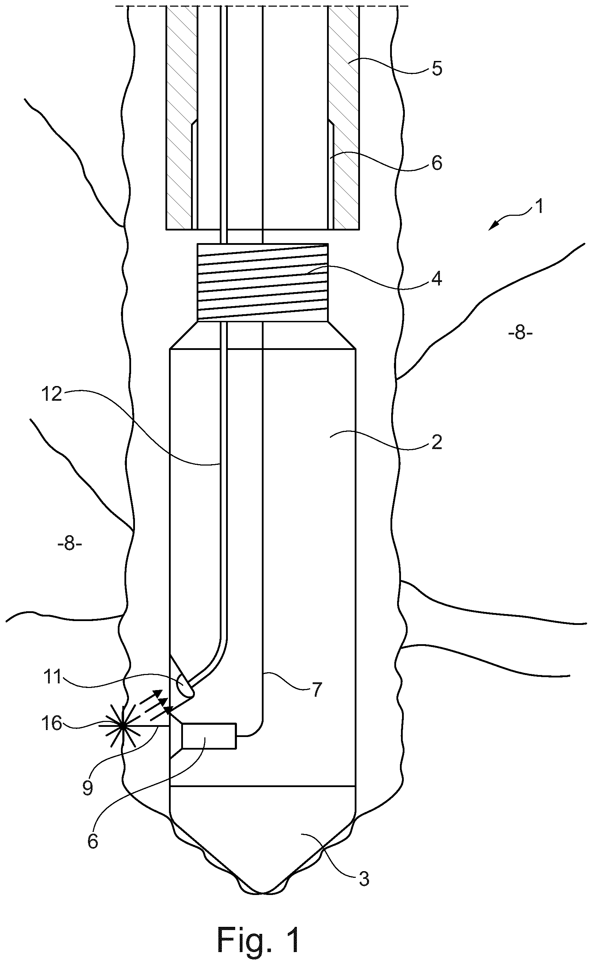

Figure 1 shows a first embodiment 1 of a device according to the invention. The device 1 has a head with an elongate, cylindrical body 2, a penetration cone 3 arranged on one end of the body 2 and a threaded portion 4 arranged on the other end of the elongate body 2.

A tubular driving rod 5 with an inner thread 6 is shown disconnected from the head 2, 3, 4 for clarity. During use the tubular driving rod 5 will be screwed onto the threaded portion 4 of the head 2, 3, 4.

A laser device 6 is arranged in the head 2, 3, 4 and is controlled via a cable 7, which runs through the string of driving rods 5 towards the surface of a soil layer 8. The laser device 6 emits a laser beam 9 in radial direction of the cylindrical body 2 onto a point 10 of a material present in the soil layer 8.

The laser beam 9 will generate a plasma, which will emit light which is received by a lens 11 of an optical cable 12 which also runs through the tubular driving rod 5 towards the surface of the soil layer 8.

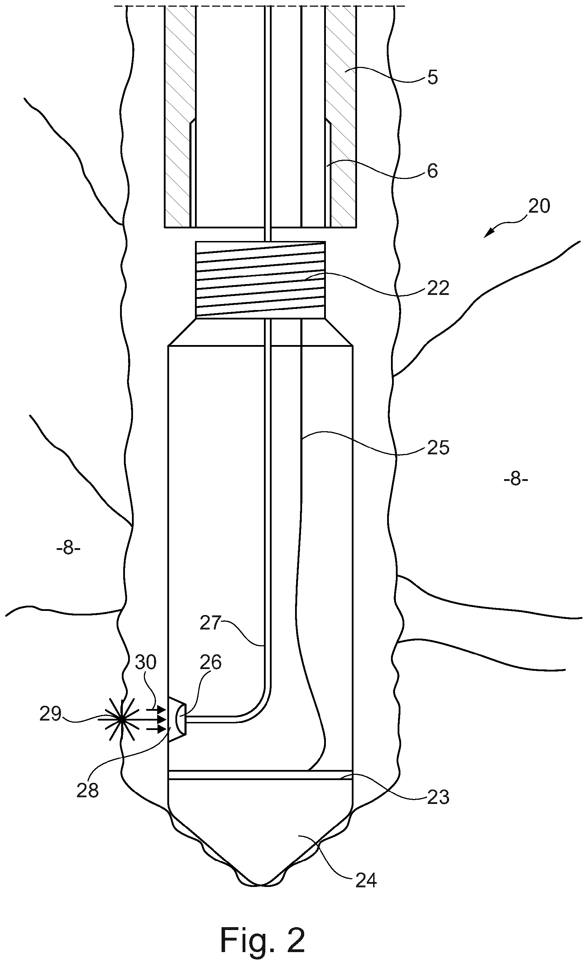

Figure 2 shows a second embodiment 20 of a device according to the invention.

The device 20 has a head with a cylindrical body 21, a threaded end 22 for connection to a tubular driving rod 5. On the opposite end of the cylindrical body 21, a force sensor 23 and a penetration cone 24 is arranged. The force sensor 23 is connected to a cable 25 which runs along the driving rod 5 towards the surface. The force sensor 23 registers the force required to penetrate the soil layer 8 with the penetration cone 24.

The head 21, 22, 23, 24 is furthermore provided with an optical path comprising a lens 26 and an optical cable 27 which runs to the surface of the soil layer 8. The optical path is used to send a laser beam 28 onto a spot 29 of the soil layer 8 and to receive light 30 emitted from the spot 29.

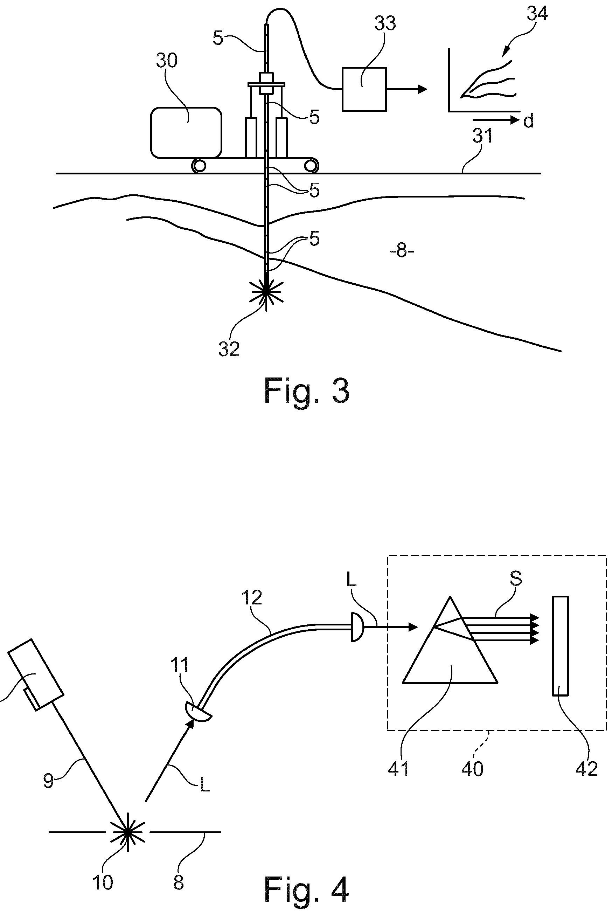

Figure 3 shows a schematic view of an embodiment of a method according to the invention. According to the method a driving rig 30 is provided on the surface 31 of a soil layer 8. The driving rig 30 drives a string of tubular driving rods 5 with at the lower end a head as described in figures 1 and 2, into the soil layer 8.

During the penetration of the head into the soil layer 8 repeated measurements are performed by firing the laser beam 32 onto the material of the soil layer 8. These measurements are processed by a controller 33 resulting in a diagram 34 showing geological information of the soil layer 8 depending on the depth d.

Figure 4 show schematically the functionality of the device 1 of figure 1. The laser device 6 fires a laser beam 9 onto a position 10 at the soil layer 8. Light L emitted from the position 10 is received by a lens 11 and optical cable 12 which runs to the surface 31, where a spectrometer 40 is provided.

The spectrometer 40 separates the light L into spectral components S with a prism 41, where after the spectral components S are measured by a detector 42, such as a CCD chip.

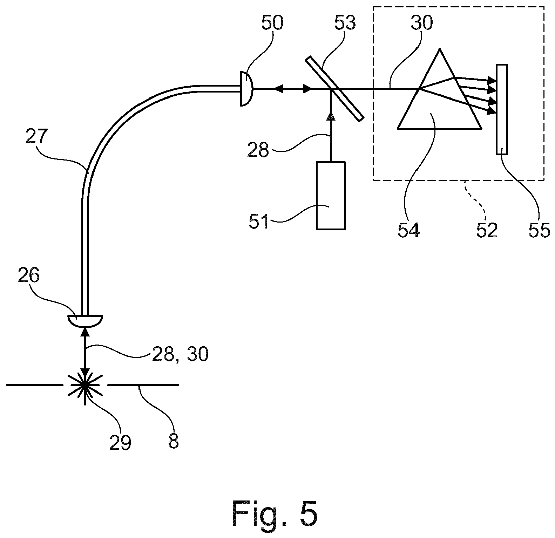

Figure 5 shows schematically the functionality of the device of figure 2. An optical path is provided by a lens 26, an optical cable 27 and a second lens 50. The optical path 26, 27, 50 runs from the head of the device towards the surface 31 of the soil layer 8.

A laser device 51 generates a laser beam 28 which is fed to the optical path by an angled two-way mirror 53. The laser beam 28 runs through the optical cable 27 and is focused on the spot 29 where a plasma is generated from the material of the soil layer 8. The emitted light 30 is received by the optical path and transported along the optical cable 27 to a spectrometer 52, which separates the light 30 into spectral components S with a prism 54, and are then measured by a detector 55.

## Claims

1. Device for obtaining geological and/or chemical information of a soil layer, which device comprises
- a string of driving rods; and
- a head arranged at the end of the string of driving rods, such that the head can be driven into a soil layer by the string of driving rods,
which head comprises an elongate, preferably cylindrical, body having on one elongate end a penetration cone and having on the other elongate end a coupling, such as a threaded end, for coupling with the string of driving rods
**characterized by**
- a device for laser-induced breakdown spectroscopy and / or Raman spectroscopy having at least a laser device for generating a laser beam to be directed on to a material, an optical path for receiving light emitted from the material, and a spectrometer for separating and measuring spectral components of the received light transported along the optical path;
wherein the laser beam exits the device at the head and wherein the optical path starts at least adjacent to the position where the laser beam exits the head.

2. Device according to claim 1, wherein the position where the laser beam exits the head is on the elongate circumferential surface.

3. Device according to claim 1 or 2, wherein the optical path comprises an optical fiber starting at the head and running through the string of rods.

4. Device according to claim 3, wherein the laser beam is guided along the optical fiber towards the head.

5. Device according to any of the preceding claims, wherein a force sensor is arranged between the penetration cone and the body.

6. Method for obtaining geological information of a soil layer, which method comprises the steps of:
- providing a driving rig positioned on the surface of a soil layer;
- providing the device according to any of the preceding claims;
- driving the head of the device with the string of driving rods into the soil layer by driving the string of driving rods with the driving rig;
- while monitoring the depth of the head into the soil layer repeatedly operating the device for laser-induced breakdown spectroscopy and / or Raman spectroscopy and registering the measured spectral components in combination with the depth of the head.

7. Method according to claim 6 and claim 5, wherein the registered force of the force sensor is monitored and registered in combination with the depth of the head.
